(19) Europäisches Patentamt
European Patent Office
Office européen des brevets

(11) **EP 1 716 387 B1**

(12) **FASCICULE DE BREVET EUROPEEN**

(45) Date de publication et mention
de la délivrance du brevet:
**19.08.2009 Bulletin 2009/34**

(51) Int Cl.:
**G01B 7/14** *(2006.01)* **G01D 5/20** *(2006.01)*

(21) Numéro de dépôt: **05701533.1**

(22) Date de dépôt: **17.01.2005**

(86) Numéro de dépôt international:
**PCT/EP2005/050178**

(87) Numéro de publication internationale:
**WO 2005/071353 (04.08.2005 Gazette 2005/31)**

(54) **DISPOSITIF DE MESURE DE DISTANCE.**

ABSTANDSMESSEINRICHTUNG

DISTANCE MEASURING DEVICE

(84) Etats contractants désignés:
**AT BE BG CH CY CZ DE DK EE ES FI FR GB GR HU IE IS IT LI LT LU MC NL PL PT RO SE SI SK TR**

(30) Priorité: **16.01.2004 EP 04075045**

(43) Date de publication de la demande:
**02.11.2006 Bulletin 2006/44**

(73) Titulaires:
• **Nomics, société anonyme**
**4031 Liège (Angleur) (BE)**
• **Logistique Spatiale Wallonne**
**4031 Angleur-Liège (BE)**

(72) Inventeurs:
• **ANSAY, PIERRE,**
**Nomics (Société Anonyme)**
**4000 Liège (BE)**

• **POIRRIER, ROBERT,**
**Nomics (Société Anonyme)**
**4000 Liège (BE)**
• **BECKERS, BERNARD,**
**Nomics (Société Anonyme)**
**4000 Liège (BE)**

(74) Mandataire: **Quintelier, Claude**
**Gevers & Vander Haeghen**
**Intellectual Property House**
**Holidaystraat 5**
**Brussels Airport Business Park**
**1831 Diegem (BE)**

(56) Documents cités:
DE-A- 4 114 398 FR-A- 2 692 979
US-A- 4 665 361 US-A- 4 843 259
US-A1- 2002 115 944 US-B1- 6 234 654

**Description**

L'invention concerne un dispositif de mesure de distance comprenant un émetteur et un récepteur, ledit émetteur étant agencé pour produire un champ magnétique à l'aide d'un circuit résonant ayant une fréquence de résonance, ledit récepteur étant agencé pour capter à ladite fréquence de résonance le champ magnétique émis par l'émetteur et convertir l'intensité du champ magnétique capté en un premier signal ayant une valeur d'énergie, ledit émetteur étant agencé pour produire ledit champ magnétique par intermittence, chaque émission ayant une énergie prédéterminée, ledit récepteur étant connecté à un détecteur agencé pour déterminer un signal de mesure de distance représentant la distance entre l'émetteur et le récepteur.

L'invention concerne également un détecteur de troubles du sommeil comprenant un dispositif de mesure de distance.

Un tel dispositif est connu de la demande de brevet DE 41 14 398. Suivant le dispositif connu, l'intensité du champ magnétique capté donne une mesure de la distance entre l'émetteur et le récepteur et peut de cette façon être utilisé pour mesurer une distance entre deux points. Pour obtenir cette distance le premier signal est amplifié de façon sélective.

Un inconvénient du dispositif connu est qu'il n'est pas adapté pour mesurer de façon fiable et précise des distances de plus de quelques centimètres sans devoir utiliser un champ magnétique d'intensité élevée. De plus l'amplification sélective du premier signal n'étant pas précisée, elle ne permet pas une détermination précise de la distance, en particulier lorsque le premier signal comporte une quantité de bruit et de signaux parasites. Pour cette raison ce dispositif connu n'est pas apte à mesurer de façon fiable des mouvements de la bouche d'un être vivant dans des applications où une grande résolution est requise. En effet des champs magnétiques à puissance élevée ne sont pas appropriés à être utilisés fréquemment sur des êtres vivant sans porter atteinte à la santé de cette être vivant.

L'invention a pour but de réaliser un dispositif de mesure de distance qui est capable de mesurer de façon très précise des distances, en particulier sur le corps humain, sans devoir utiliser une valeur de champ magnétique qui serait trop forte pour le corps humain.

A cette fin un dispositif suivant l'invention est caractérisé en ce que ledit détecteur est agencé pour déterminer ledit signal de mesure de distance par corrélation dudit premier signal avec un deuxième signal prédéterminé ayant une forme d'onde représentative d'un signal à capter par le récepteur, ledit deuxième signal comportant une fenêtre temporelle ayant une durée prédétermine et comprenant au moins une sous-période initiale, une sous-période intermédiaire et une sous-période finale, ledit deuxième signal étant un signal alternatif synchronisé avec le premier signal et dont l'amplitude est atténuée durant les périodes initiales et finales et substantiellement au maximum durant la période intermédiaire. L'utilisation d'un signal alternatif dont l'amplitude est atténuée durant la sous-période initiale et finale permet de considérablement réduire le bruit et les parasites apparaissant dans des gammes de fréquence éloignées de la fréquence de résonance. Le fait que l'amplitude soit substantiellement au maximum durant la période intermédiaire, c'est à dire celle où le premier signal atteint sa valeur maximale, permet de considérablement réduire le bruit et les parasites dans les gammes de fréquence très proches de la fréquence de résonance tout en exploitant au maximum l'amplitude de ce signal durant cette sous-période intermédiaire et donc de pouvoir travailler avec des champs magnétiques dont la puissance reste faible et donc sans dommage pour le corps humain.

Une première forme de réalisation préférentielle d'un dispositif suivant l'invention est caractérisée en ce que ledit détecteur est agencé pour réaliser ladite corrélation par multiplication et intégration avec ledit deuxième signal, lequel deuxième signal est formé par ladite forme d'onde représentant une forme d'onde sinusoïdale en synchronisation avec le premier signal elle-même multipliée par une fenêtre de Tukey à coefficient d'effilage réduit. Ceci permet un rejet de bruit et des parasites en-dehors de la fréquence de détection.

Une deuxième forme de réalisation préférentielle d'un dispositif suivant l'invention est caractérisée en ce que ledit émetteur est logé dans un boîtier et agencé pour produire ledit champ magnétique à l'extérieur dudit boîtier avec une puissance inférieure à 1mTesla, de préférence inférieure à 1µTesla. Cette forme de réalisation est particulièrement adaptée au corps humain.

Un détecteur de troubles de sommeil suivant l'invention est caractérisé en ce que ledit dispositif est monté sur un support agencé pour être appliqué sur la tête d'un être vivant de telle façon à mesurer des mouvements de la bouche.

L'invention sera maintenant décrite plus en détails à l'aide des dessins qui représentent une forme de réalisation préférentielle d'un dispositif suivant l'invention ainsi que d'un détecteur de troubles du sommeil comprenant un dispositif de mesure de distance suivant l'invention. Dans les dessins :

la figure 1 illustre la structure de base du dispositif de mesure;
la figure 2 illustre l'excitation de l'émetteur ainsi que le signal émis par l'émetteur et le signal reçu par le récepteur;
la figure 3 illustre diverses topologies d'excitation d'un circuit résonant série formant l'émetteur;
la figure 4 illustre deux exemples du circuit du récepteur;
la figure 5 illustre le principe de la détection par corrélation du signal avec un modèle du signal à détecter;

les figures 6 et 7 montrent un exemple d'un signal reçu et du signal obtenu après détection par corrélation;

la figure 8 montre une implémentation numérique de la détection par corrélation;

la figure 9 montre un modèle correspondant a une méthode de détection généralement connue et deux modèles préférentiels, utilisables pour une détection par corrélation;

les figures 10 et 11 montrent les propriétés de rejet de bruit et des parasites de ces trois méthodes de détection par corrélation;

les figures 12 et 13 montrent le placement du dispositif sur un être vivant;

les figures 14 a), 14 b) et 15 illustrent à l'aide d'un organigramme une façon de détecter des troubles de sommeil;

la figure 16 reprend des signaux de mouvement d'une bouche;

la figure 17 illustre une clef de convolution;

la figure 18 illustre le résultat de la convolution;

la figure 19 représente une période de ronflement;

la figure 20 montre un tableau utiliser pour déterminer les apnées;

la figure 21 illustre la position des capteurs pour détecter la maladie de Parkinson;

les figures 22 a et b montrent les modèles correspondants aux techniques classiques de détection synchrone et de « lock-in », alors que les figures 22 c et d montrent deux exemples de modèles tels qu'utilisés dans la présente invention; et

la figure 23 montre la réponse fréquentielle des modèles repris dans les figures 22 a à d.

Dans les dessins une même référence a été attribuée à un même élément ou à un élément analogue.

Le dispositif de mesure de distance utilise un capteur de distance robuste à usage physiologique. Son principe magnétique offre de nombreuses possibilités de mesure, puisqu'il n'est pas influencé par la présence de vêtements ou l'appui contre un oreiller ou un matelas (dans les applications en cours de sommeil), ni par l'état d'agitation ou de transpiration de l'être vivant sur lequel il est appliqué. Le champ magnétique nécessaire à la mesure est extrêmement réduit. L'émission de champ est périodique, à la cadence de mesure, avec un rapport cyclique extrêmement faible, alors que son amplitude maximale atteint une valeur inférieure à 1mTesla, de préférence inférieure à $1\mu$Tesla, voir même de l'ordre de 0,4 $\mu$Tesla, réputée non nocive pour la santé en cas d'exposition continue.

Le capteur est conçu pour fonctionner en couplage magnétique lâche, ce qui signifie que l'émetteur influence le récepteur, mais que l'atténuation entre l'émetteur et le récepteur ne permet pas au récepteur d'influencer l'émetteur en retour. La méthode de détection est performante et basée sur une détection par corrélation qui permet, malgré le niveau très réduit du champ magnétique émis, d'étendre la gamme de mesure dans un rapport de distance allant jusqu'à 5, voire 6 fois supérieurs aux dispositifs connus. Des rapports de distance plus élevés peuvent être atteints en augmentant le champ émis pour limiter l'influence des parasites. Selon les applications, le capteur peut être réalisé avec des composants de très petite taille pour des distances allant de 5 à 30 mm, ou des inductances de taille moyenne (moins de 10mm de diamètre et moins de 15mm de long) pour une gamme de mesure de 7 à 35 cm en conservant un champ magnétique d'amplitude maximale inférieure à $1\mu$Tesla. Les caractéristiques de ce capteur permettent de réaliser aisément un système ergonomique à placement aisé par le sujet lui-même, de sorte qu'il est utilisable dans les applications ambulatoires.

Plusieurs mesures de distance par rapport à la même référence sont possibles en utilisant un seul circuit d'émission magnétique. Une excitation bilatérale du circuit d'émission magnétique permet en outre d'utiliser ce dispositif pour la transmission d'informations à faible débit et à courte distance. La mesure simultanée de la distance de communication trouve notamment des applications de sécurité. L'obtention de performances plus élevées encore, tant en mesure qu'en fiabilité de transmission, est possible en donnant un caractère pseudo-aléatoire au champ magnétique émis.

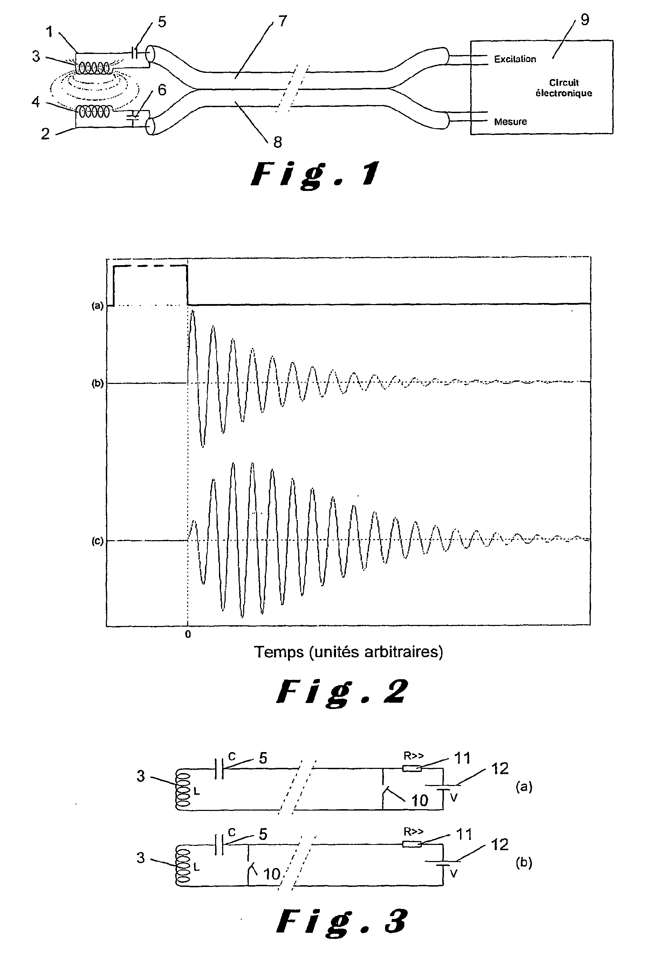

La figure 1 illustre un exemple d'une structure de base d'un dispositif suivant l'invention. Le dispositif comporte un émetteur 1 et un récepteur 2 destinés à être placé à distance l'un de l'autre. L'émetteur comporte une inductance 3 connectée en série avec un condensateur 5, alors que dans le récepteur l'inductance 4 et le condensateur 6 sont connectés en parallèle. L'émetteur 1, respectivement le récepteur 2 sont relié à l'aide d'un câble 7, respectivement 8, à une unité 9 de conditionnement et de mesure. L'unité 9 comprenant un détecteur et un circuit d'excitation comme il sera décrit ci-dessous. Les capteurs utilisés utilisent la propriété qu'a un circuit résonant d'en exciter un autre accordé sur la même fréquence, au travers de leur inductance (3,4) mutuelle. L'utilisation de circuits résonants plutôt que de simples inductances améliore significativement à la fois les performances du circuit d'excitation et la sensibilité du capteur. L'utilisation d'une connexion simultanée de l'émetteur et du récepteur au même circuit électronique permet de simplifier le dispositif en s'affranchissant des erreurs de synchronisation. Dans ce cas, pour des questions de confort, les câbles sont généralement rassemblés sur la majeure partie de leur longueur, pour se séparer seulement à l'approche des éléments de mesure. Toutefois, les circuits d'émission et de réception peuvent être désolidarisés sans porter atteinte au principe de mesure.

Afin de limiter le champ magnétique moyen émis, et d'éviter ainsi l'induction à long terme d'effets nocifs ou même simplement gênants pour le patient, et afin de réduire la consommation électrique, le circuit résonant d'émission est excité ponctuellement à chaque prise de mesure, comme illustré à la figure 2. Une impulsion de courant, comme illustrée en 2a, est produite par le circuit d'excitation afin d'exciter l'émetteur à l'aide d'un courant électrique. Le courant circulant dans l'inductance émettrice 3, et qui est proportionnel au champ magnétique émis, prend une forme légèrement différente selon le mode d'excitation adopté, mais d'allure générale telle que représentée à la figure 2b. La tension recueillie au récepteur 2, en cas de compatibilité des circuits résonants, se présente comme à la figure 2c. La résonance se produit suite à la fermeture du circuit résonant au temps 0, après une période d'initialisation préalable montrée en (a), qui a pour but de créer une tension aux bornes de la capacité et/ou un courant dans l'inductance.

L'enveloppe de ce signal dépend du facteur de qualité des circuits résonants utilisés. En cas de mauvaise compatibilité des circuits résonants, cette enveloppe peut se modifier de manière significative, avec l'apparition éventuelle d'un phénomène de battement. La valeur maximale de la tension observée au récepteur varie en fonction de la distance entre inductance d'émission 3 et inductance de réception 4 selon la relation :

$$V = (\alpha/d^3) + \beta \quad (1)$$

où $\alpha$ est le gain global de détection, en incluant l'effet des amplificateurs, et $\beta$ un décalage éventuel dû aux circuits de détection et d la distance entre l'émetteur et le récepteur.

Dans la forme préférée de l'invention, les circuits résonants sont accordés sur une fréquence de 5 à 8 kHz, de manière à maximiser le facteur de qualité des circuits résonants tout en restant en dessous des fréquences radio. L'utilisation d'inductances miniatures peut toutefois nous mener à adopter des fréquences allant jusque 50, voire 100 kHz, en fonction des possibilités du circuit de détection. La valeur et la taille des inductances de résonance sont adaptées de manière à pouvoir baser la détection sur un signal présentant de 10 à 30 périodes de résonance d'amplitude significative. Des durées inférieures dégradent les performances de détection, alors que des valeurs supérieures demandent une précision trop grande, en pratique, dans l'accord des circuits résonants.

Diverses topologies peuvent être adoptées pour le circuit d'excitation. Le choix doit tenir compte du niveau d'excitation désiré (amplitude maximale du champ magnétique), du couplage parasite existant entre les fils 7 et 8 menant de l'unité 9 à l'émetteur et au récepteur (spécialement lors de l'utilisation de câbles très fins pour le confort des personnes), et des aspects de compatibilité électromagnétique. Diverses topologies d'un circuit résonant série sont présentées à la figure 3. Le circuit d'excitation comporte lui une résistance 11, un élément de commutation ou interrupteur 10 et une source de tension 12 branchés en série au travers des conducteurs électriques 7 en série avec l'inductance et le condensateur. L'excitation s'effectue par une fermeture brève de l'interrupteur 10 durant laquelle la capacité 5 est chargée à la tension d'alimentation et un courant est créé dans l'inductance 3. Au moment de l'ouverture de l'interrupteur 10, le circuit formé par l'inductance 3 et la capacité 5 se met donc à osciller sous l'effet de ces deux conditions initiales Le principal attrait de cette méthode est l'obtention aisée de niveaux d'excitation importants ou même variables, en fonction du temps de fermeture de l'interrupteur. Les circuits illustrés à la figure 3 sont des circuits d'excitation série dit « à régénération ». L'excitation est produite par la fermeture de l'interrupteur 10, qui reste fermé tout au long de la mesure. L'ouverture de l'interrupteur permet alors à la capacité 5 de se recharger sans oscillation au travers de la résistance 11.. La forme illustrée à la figure 3a facilite le contrôle de l'interrupteur, alors que la forme 3b permet à la fois d'annuler la tension dans le câble et de rendre constant le courant dans le câble pendant la mesure. Ces formes permettent d'éviter le couplage parasite dans les câbles, puisque l'interrupteur fermé impose une tension très faible dans le câble de l'émetteur durant la mesure. De plus la forme de la figure 3a offre l'avantage que l'interrupteur est plus facile à commander puisqu'il se situe près de la source 12 présente dans l'unité 9.

L'excitation en résonance série peut se réaliser sous deux formes principales, à savoir l'excitation alternée et l'excitation simple à régénération. L'excitation alternée consiste à piloter le circuit résonant par une source de tension d'allure carrée. Une excitation est réalisée à chaque transition de la source de tension, mais le signe du champ électromagnétique émis lors d'une transition descendante est inversé à celui observé lors d'une transition montante. Cet effet doit impérativement être compensé au niveau du circuit de détection pour éviter des imprécisions de mesure. L'excitation simple à régénération consiste à charger lentement la capacité du circuit résonant à travers une résistance de valeur élevée pour éviter une résonance, puis de fermer l'interrupteur pendant toute la mesure pour laisser osciller librement le circuit résonant ainsi formé.

La forme préférée de l'invention utilise donc une excitation à régénération avec une capacité intégrée à l'élément d'émission (figure 3a et b). Ceci permet de maintenir une tension théoriquement nulle tout au long du câble durant la mesure. La topologie à excitation décentralisée (figure 3b) présente en plus l'avantage d'un courant constant durant la mesure.

L'émetteur est agencé pour produire un champ magnétique à l'aide du circuit résonant formé par l'inductance 3 et le condensateur 5. Le circuit résonant possède une fréquence de résonance et le récepteur est agencé pour capter à ladite fréquence de résonance le champ magnétique émis par l'émetteur et convertir l'intensité du champ magnétique capté en un premier signal ayant une valeur d'énergie. Comme illustré à la figure 2 a c'est à partir d'une impulsion fournie au circuit d'excitation que l'émetteur produit le champ magnétique. Ainsi ce champ magnétique est produit par intermittence et chaque émission a une énergie prédéterminée, notamment déterminée par la valeur de l'inductance et du condensateur.

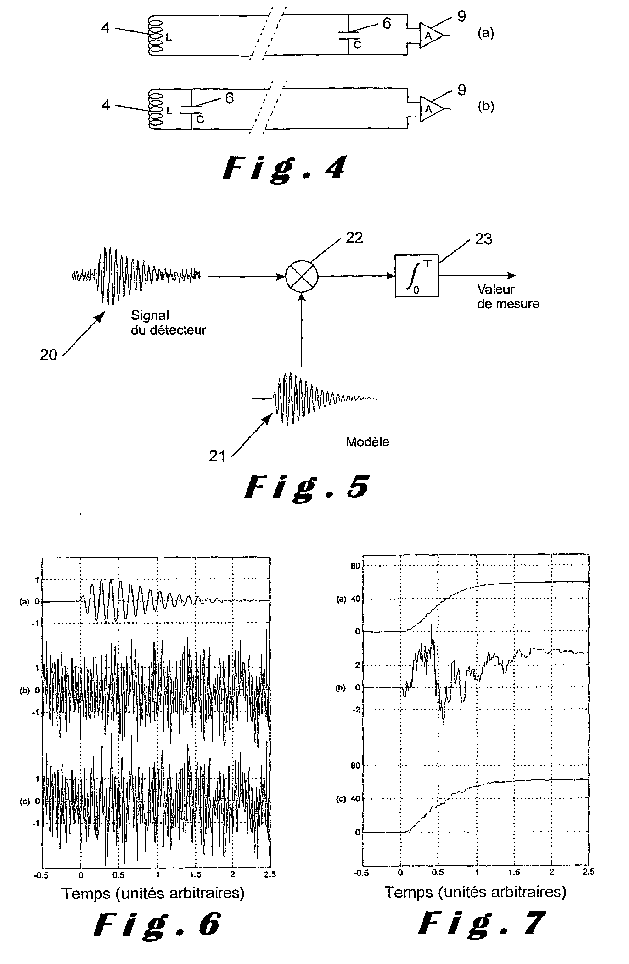

La figure 4 illustre deux exemples du circuit du récepteur 2, notamment (4a) à capacité intégrée et (4b) à capacité éloignée. Le récepteur est agencé pour capter à la fréquence de résonance le champ magnétique émis par l'émetteur et convertir l'intensité du champ magnétique capté en un premier signal ayant une valeur d'énergie qui représente l'énergie captée par le récepteur. Le choix de la méthode de détection, c'est à dire d'utilisation de la tension observée pour obtenir une image de la distance, est essentiel pour minimiser les valeurs de champ requises, et ainsi les effets néfastes possibles sur les personnes. La détection est réalisée par corrélation comme il sera décrit ci-dessous.

Si l'on respecte une distance minimale de mesure où le couplage entre les inductances d'émission 3 et de réception 4 est suffisamment lâche, on peut considérer que la forme d'onde du signal reçu n'est modifiée qu'en amplitude en fonction de la distance. On peut donc utiliser l'ensemble de cette forme d'onde pour améliorer la détection. Une méthode connue procède par l'implémentation d'une détection synchrone. Cette méthode consiste à multiplier le signal reçu par 1 lorsque la forme d'onde théorique est réputée positive, et par -1 lorsqu'elle est réputée négative, puis d'intégrer le signal ainsi obtenu. Une autre méthode connue procède par l'implémentation d'une détection de type » lock-in », qui consiste à multiplier le signal reçu par une sinusoïde synchronisée avec le signal reçu, de manière telle que les alternances positives de la sinusoïde correspondent aux alternances positives du signal à recevoir, et les alternances négatives de la sinusoïde correspondent aux alternances négatives du signal à recevoir, puis à intégrer le signal ainsi obtenu. Ces méthodes de détection synchrone ou « lock-in » sont représentées sous forme analogique, mais peuvent tout aussi bien s'effectuer par voie numérique. Cette détection synchrone est une forme de réalisation d'une détection par corrélation du signal capté par le récepteur avec un deuxième signal prédéterminé ayant une forme d'onde représentative d'un signal à capter par le récepteur. Le résultat issu de cette corrélation constitue alors un signal de mesure de distance représentant la distance entre l'émetteur et le récepteur. La détection par corrélation consiste à multiplier le signal reçu par un modèle, éventuellement modifié, de la forme d'onde théorique, puis d'intégrer le signal ainsi obtenu. La valeur obtenue à la fin du temps d'intégration est la mesure recherchée. Cette méthode générique a l'avantage d'intégrer comme cas particulier les méthodes connues de détection synchrone, de « lock-in » et de filtrage adapté, selon la forme donnée au modèle de détection. Le principe général est représenté à la figure 5, et son efficacité pour la détection d'un signal bruité est illustrée à la figure 6. Le signal capté 20 est multiplié (22) avec un deuxième signal 21 et le résultat de cette multiplication est intégré (23) pour fournir la valeur de mesure. La figure 6a montre un exemple d'un deuxième signal (21), la figure 6b un exemple du bruit seul, à un niveau relatif, par rapport au signal capté, correspondant à des conditions typiques de détection aux deux tiers de la gamme de mesure de signaux reçus et la figure 6c représente le cas réel où le signal capté est combiné avec le bruit. La figure 7 représente le résultat de l'intégration lors d'une détection par corrélation. Les références a à c dans la figure 7 correspondent à celles de la figure 6. On constate donc dans la figure 7c que le signal de mesure augmente pour atteindre un niveau correspondant à distance entre émetteur et récepteur.

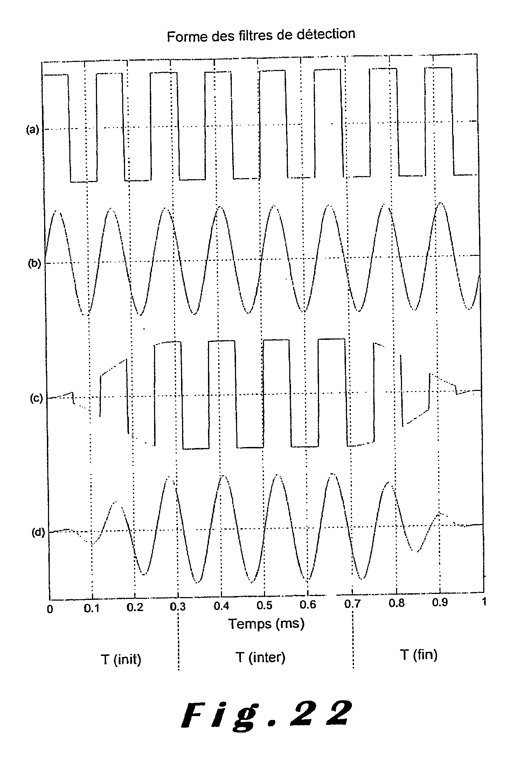

Les méthodes connues de détection synchrone ou « lock-in » peuvent être réalisées sous forme de détection par corrélation en utilisant comme deuxième signal les modèles présentés à la figure 22 a et b, respectivement. La présente invention consiste à utiliser, en tant que deuxième signal, un signal alternatif synchronisé avec le premier signal issu du signal capté par le récepteur. Ce signal alternatif comporte une fenêtre temporelle ayant une durée prédéterminée (T) comme illustré à la figure 22 c et d. La fenêtre temporelle comporte au moins une sous-période initiale T(init), une sous-période intermédiaire T(inter) et une sous-période finale T(fin). Le modèle possède une amplitude qui est atténuée dans les sous-périodes T(init) et T(fin), et une amplitude substantiellement au maximum durant la sous-période intermédiaire T(inter). Le modèle de la figure 22 c correspond à une adaptation selon la présente invention de la méthode de détection synchrone. Le modèle de la figure 22 d correspond à une adaptation selon la présente invention de la méthode de détection « lock-in ».

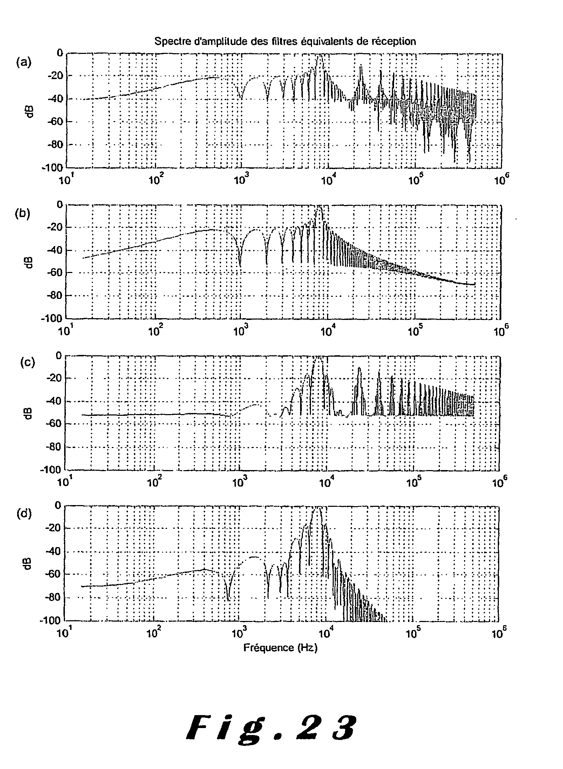

La figure 23 illustre l'avantage de l'invention sur les méthodes connues au travers des caractéristiques fréquentielles de détection, les figures 23 a à d correspondant aux modèles de la figure 22 a à d, respectivement. Les figures 23 c et d montrent clairement que l'atténuation du bruit et des parasites présents en-dehors de la bande utile du signal, ici fixée à 8kHz, est supérieure à celle obtenue avec les méthodes connues.

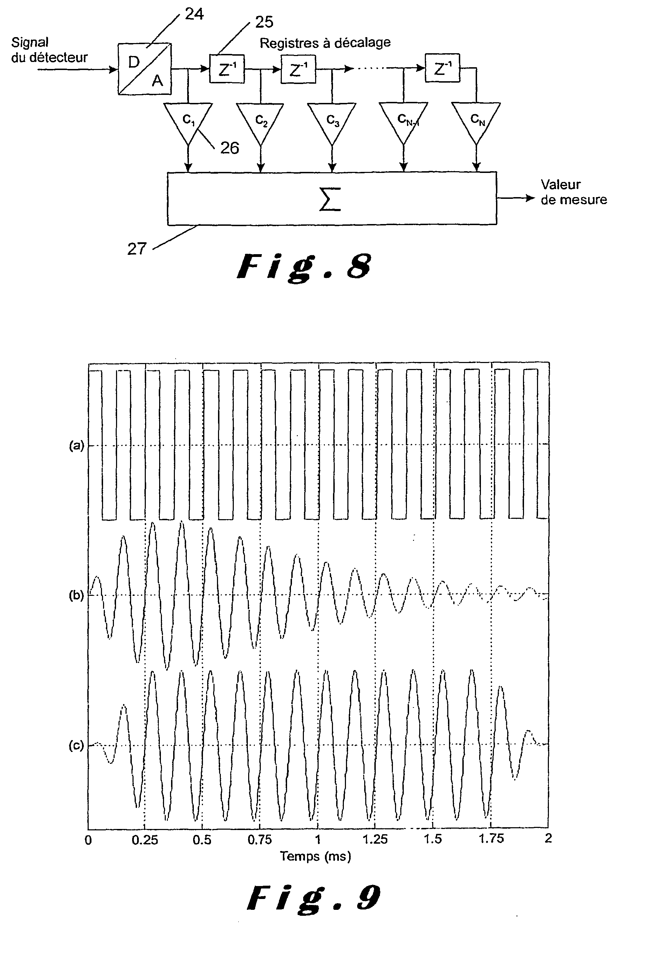

Le filtrage adapté, qui est une forme de réalisation d'un détection par corrélation, est une technique utilisée en télécommunications, et est réputée fournir le meilleur rapport signal à bruit sous l'hypothèse d'un bruit blanc gaussien. Des expérimentations ont montré que cette méthode est également excellente dans le cadre de cette invention. Elle est par ailleurs précise si l'on s'interdit l'utilisation de filtres adaptatifs qui posent des problèmes de calibrage des mesures. La technique du filtre adapté consiste à appliquer au signal reçu un filtre dont la réponse pulsionnelle est l'image renversée de la forme d'onde théorique du signal à recevoir, puis d'échantillonner le signal obtenu à un moment précis correspondant à l'alignement parfait du signal utile et du filtre. En d'autres termes, on peut aussi dire que le résultat de la mesure est la valeur de corrélation entre la portion du signal où l'on sait se trouver le signal utile, et la forme d'onde théorique de ce signal utile. Etant donné la complexité de la forme d'onde à réaliser, ce filtrage est réalisé en numérique. L'application numérique de ce principe est représentée à la figure 8. Le signal reçu est converti (24) en numérique après échantillonnage et ses valeurs mémorisées sur N échantillons dans des registres à décalage 25. Ces échantillons sont ensuite multipliés (26) par les coefficients $c_i$ représentant le modèle de détection, et les résultats sont additionnés entre eux pour fournir le signal de mesure. Par intégration dans la présente description on entend donc tant sa signification mathématique que sa réalisation numérique.

La figure 9 montre des modèles utilisables pour la détection par corrélation. A titre de référence, le modèle (fig.9a) du deuxième signal pour une détection synchrone est indiqué. Le modèle pour une détection à filtrage adapté (fig.9b) est la forme d'onde théorique elle-même, qui représente le signal à capter tel qu'obtenu en l'absence de perturbations. Cette forme d'onde possède les caractéristiques répondant à la présente invention. Le modèle (fig.9c) utilisé dans une forme préférée de l'invention est une onde sinusoïdale en synchronisation avec la forme d'onde théorique, multipliée par une fenêtre de Tukey à coefficient, d'effilage (*tapper factor*) réduit (typiquement de l'ordre de 0,2 à 0,4) pour adoucir les transitions. Le principe de la fenêtre de Tukey est décrit dans Harris, F.J. « On the use of windows for harmonic analysis with the discrete Fourier transform" et publié dans Proceedings of the IEEE. Vol. 66, January 1978, pages 66 à 67.

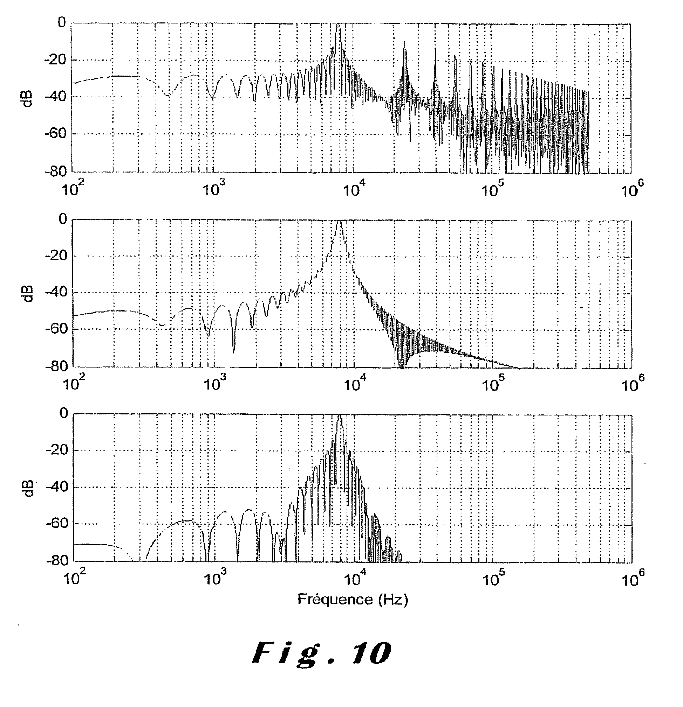

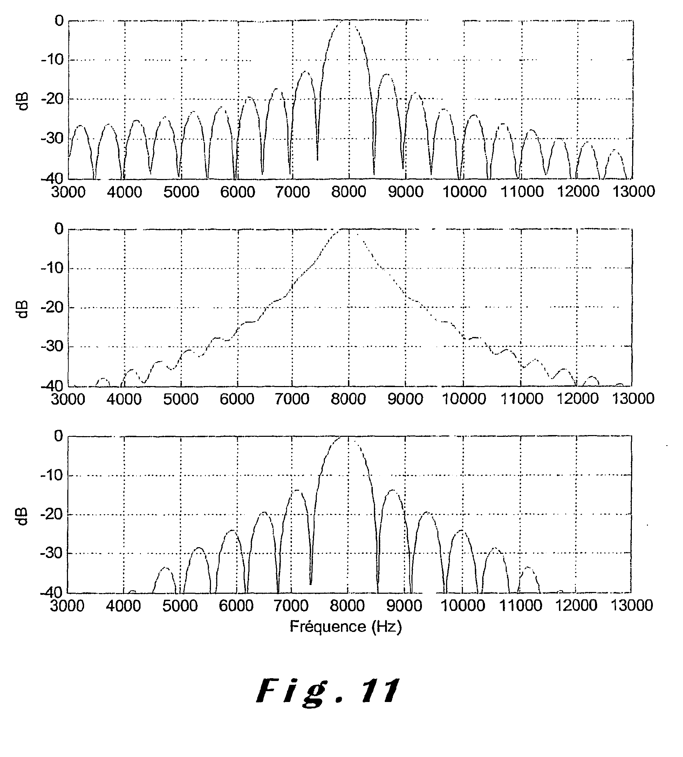

L'avantage de ce modèle tient dans les propriétés de rejet de bruit et des parasites en-dehors de la fréquence de détection. Les caractéristiques spectrales de ces trois méthodes sont représentées aux figures 10 et 11, respectivement. La figure 10 montre le spectre global de détection des différents modèles de détection de la figure 9: détection synchrone (en haut) à titre de référence, filtre adapté (au milieu) et filtre sinusoïdal à fréquence adaptée (en bas). La figure 11 montre le spectre de détection aux alentours de la fréquence de résonance des différents modèles de détection de la figure 9: détection synchrone (en haut) à titre de référence, filtre adapté (au milieu) et filtre sinusoïdal à fréquence adaptée (en bas). Le filtre adapté se montre plus efficace que la détection synchrone en-dehors de la bande passante, répondant ainsi aux caractéristiques de la présente invention, mais moins efficace pour les fréquences proches de celle de résonance. Le modèle sinusoïdal synchrone se montre globalement le plus efficace, prouvant ainsi son avantage et l'utilité de la présente invention.

L'obtention du modèle adéquat pour la détection avec un capteur particulier s'obtient lors d'une procédure de calibrage consistant à placer les deux éléments du capteur à une distance raisonnablement faible, de sorte à obtenir un signal de bonne qualité. La qualité de réception est encore améliorée en prenant la moyenne de ce signal sur plusieurs mesures. La forme d'onde obtenue peut ainsi être utilisée pour dériver, de manière triviale, chacun des modèles de forme d'onde présentés.

Performances obtenues avec un capteur optimisé pour un fonctionnement entre 2,5 et 10 cm :

| Distance de référence | 30 mm | 50 mm | 80 mm | 100 mm |
|---|---|---|---|---|
| Précision | 0,3 % | 0,5 % | 0,2 % | 0,1 % |
| Ecart-type dû au bruit | 0,003 mm | 0,05 mm | 0,30 mm | 0,73 mm |

Performances obtenues avec un capteur optimisé pour un fonctionnement entre 7,5 et 22 cm :

| Distance de référence | 75mm | 115 mm | 155 mm | 195 mm | 230 mm |
|---|---|---|---|---|---|
| Précision | 0,3% | 0,7% | 0,1% | 0,5% | 1,8% |
| Ecart-type dû au bruit | 0,039 mm | 0,12 mm | 0,28 mm | 0,56 mm | 0,91 mm |

Le stockage des formes d'onde du filtre adapté dans une mémoire placée dans le connecteur du capteur est utilisé pour rendre indépendantes le calibrage du capteur et du circuit de mesure, et faire ainsi en sorte que n'importe quel capteur (lors d'un remplacement, par exemple) puisse être branché sur le circuit en obtenant les mêmes résultats. Une autre méthode de stockage est dans la mémoire du microcontrôleur placé sur le circuit de mesure. Mais dans ce cas, il faut refaire un calibrage à chaque fois que l'on change de capteur.

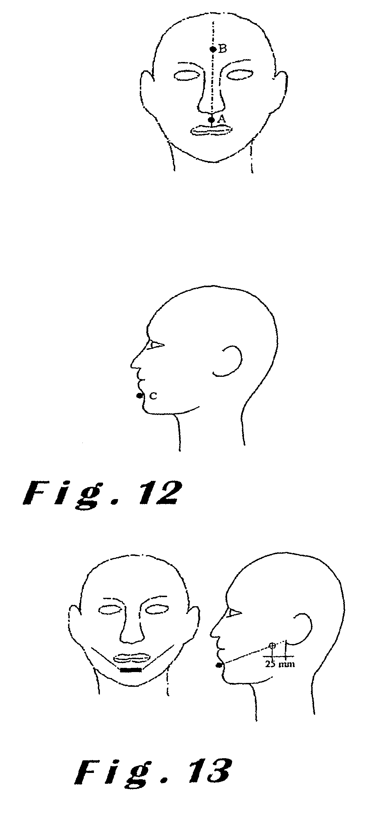

Diverses applications du dispositif de mesure suivant l'invention seront décrites ci-dessous. Il faut toutefois noter que l'usage du dispositif de mesure suivant l'invention est préférentielle, mais que ces applications peuvent également être réalisées avec d'autres dispositifs de mesure. Une application du dispositif de mesure suivant l'invention est celle à un détecteur de troubles de sommeil. Le dispositif est alors monté sur un support agencé pour être appliqué sur la tête d'un être vivant de telle façon à mesurer des mouvements de la bouche. Les paramètres de fonctionnement du capteur sont ajustés pour produire un bruit de mesure crête-à-crête inférieur à 1 mm pour des ouvertures de bouche jusqu'à 5cm par rapport à la position bouche fermée. La référence de la mesure peut être placée n'importe où le long de la ligne médiane du visage, au-dessus de la lèvre supérieure, comme indiqué à la figure 12. Une réalisation courante de l'invention place cette référence sous le nez (A). Une autre réalisation courante place cette référence sur le front (B). Alternativement, le point de référence peut être placé à l'intérieur de la bouche, sur les dents, le palais ou les gencives. Le point de mesure du capteur doit être placé de manière à suivre au mieux les mouvements de la mandibule. Dans la réalisation préférée de l'invention, il est placé dans le creux sous la lèvre inférieure (figure 13), là où les mouvements relatifs entre la structure osseuse et la peau sont les plus faibles. Alternativement, le point de mesure peut être situé à la pointe du menton, sous le menton, ou à l'intérieur de la bouche, sur les dents ou les gencives. Dans la forme préférée de l'invention, les deux éléments du capteur sont maintenus en place au moyen d'un harnais confortable, peu encombrant et de placement aisé. Le maintien du point de référence peut être effectué par n'importe quel moyen connu. Le maintien du point de mesure est plus délicat. Pour minimiser la gêne pour le patient, les structures situées près de la bouche et sur les joues doivent être très légères. D'autre part, il convient d'assurer un maintien efficace sur toute la dynamique d'ouverture sans imposer d'effort sur la mandibule qui risquerait d'influencer les mesures. Enfin, une indépendance vis-à-vis des mouvements de rotation et d'inclinaison de la tête vers l'avant ou vers l'arrière est indispensable. La forme préférée de l'invention présente une structure de capteur de forme allongée munie d'un lien imposant une faible traction depuis le creux situé sous la lèvre inférieure le long d'une ligne passant sous le lobe des oreilles, avec un point de fixation situé 25mm au moins en avant du lobe. Alternativement, un maintien complémentaire du capteur peut être assuré par une mentonnière.

Une autre possibilité de placement consiste à placer un capteur sur les paupières et un autre à proximité de l'oeil. Le but étant de mesurer les mouvements des paupières (technique utilisée également pour la détection de la somnolence). Pour étudier le Periodic Limb Movement (PLM) le capteur est placé de part et d'autre d'une articulation. Le but étant de mesurer les variations de distance de cette articulation. Une autre application serait de dire que le capteur peut être utilisé pour la transmission de données à courte distance.

La détection des troubles respiratoires du sommeil est une des applications du dispositif suivant l'invention. La surveillance des troubles respiratoires en sommeil s'applique également au cas de patients sous traitement par pression d'air positive. Ici, l'analyse réalisée du signal d'ouverture de la bouche est utilisée pour ajuster en temps réel la valeur de la pression fournie par l'appareil de PAP.

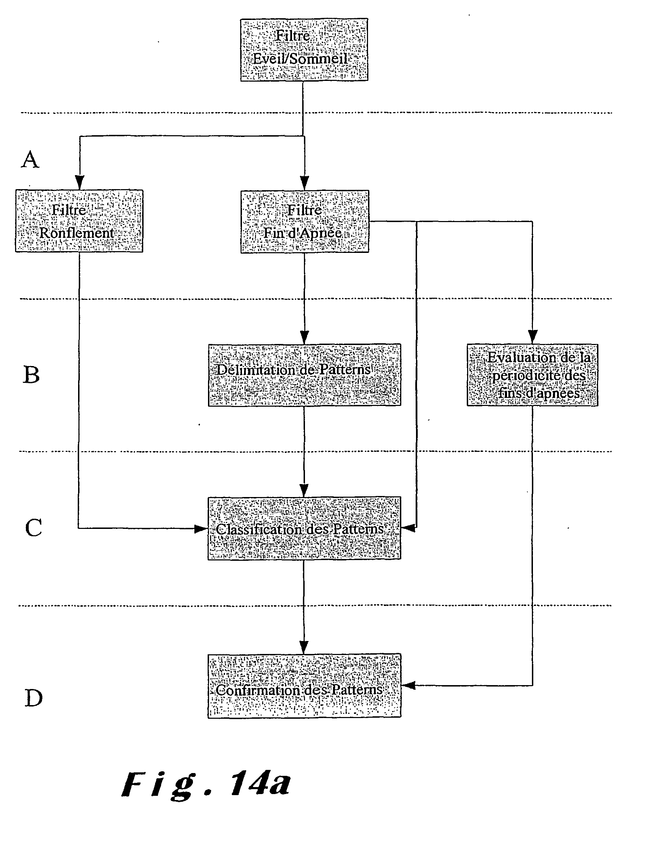

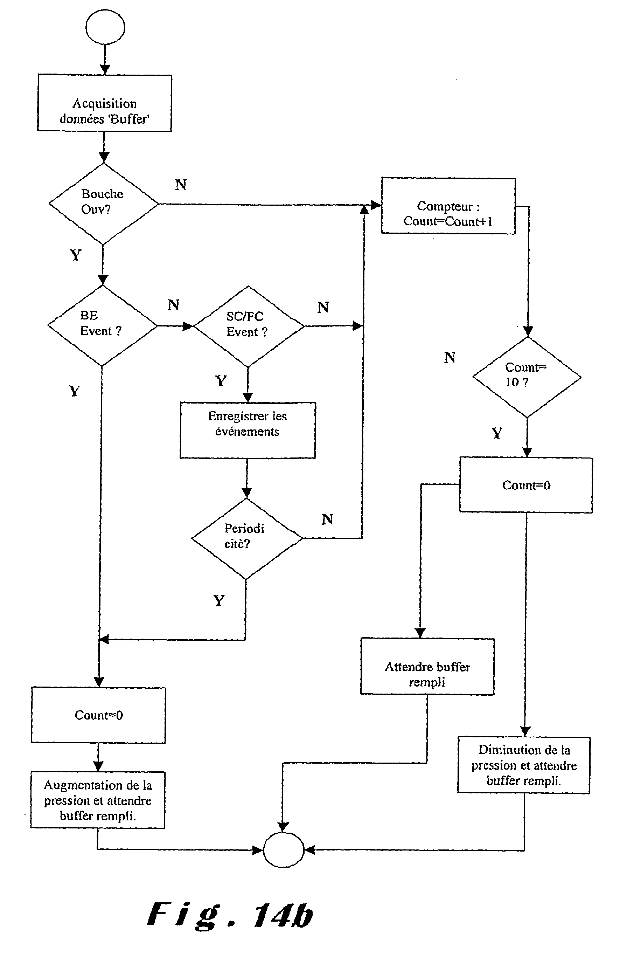

Dans cette application, des techniques de traitement de signal sont utilisées de manière à détecter tous les troubles respiratoires du sommeil. On démarre avec les données des capteurs et dérivons, par filtrage, des signaux supplémentaires. C'est la combinaison de ces signaux qui révèle les différents troubles respiratoires du sommeil: apnée centrale (ou cheynes-stokes), apnée mixte, apnée obstructive, hypopnée et résistance accrue de la voie aérienne supérieure (RAVAS ou en anglais UARS - upper airways résistance syndrome). Le diagramme schématique est décrit de ces troubles respiratoires du sommeil sont illustrés aux figures 14 a), 14 b) et 15. Le but de l'analyse est donc de déterminer si la pression appliquée est suffisante, correcte ou exagérée. Cette information est utilisée pour déterminer les ajustements de pression nécessaire et les envoyer à la CPAP.

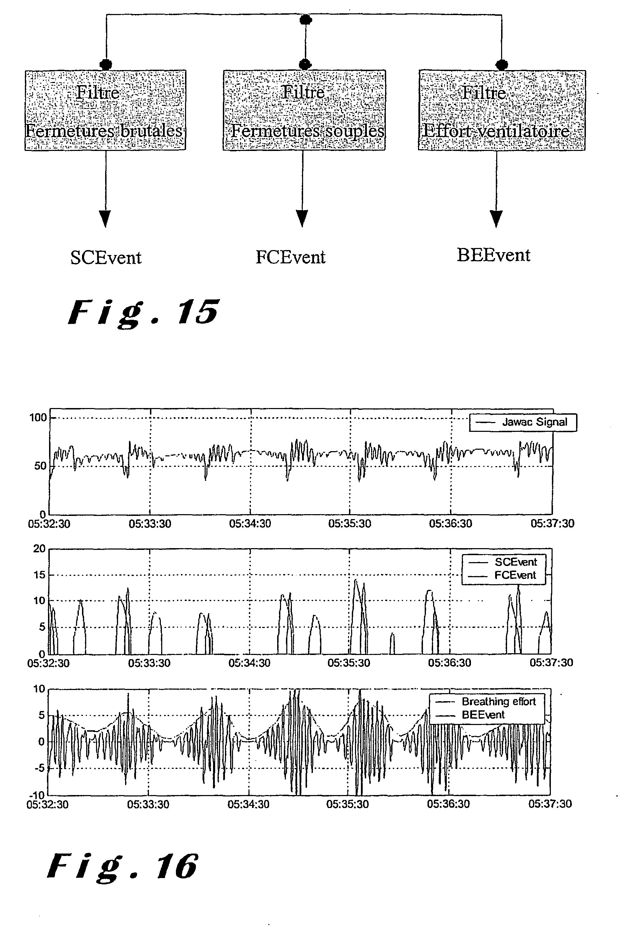

L'objectif de cette première étape (A) est d'utiliser des techniques de filtrage qui permettent de révéler les caractéristiques essentielles des apnées. Ces étapes sont précisées à la figure 15. Ces caractéristiques sont connues et décrite dans la thèse du Prof. R. Poirrier. La sortie de chaque filtre est également un signal contenant des événements. Un événement est marqueur d'une caractéristique potentielle d'une apnée et c'est la combinaison des ces événements qui sera utilisée pour la classification des différents types d'apnée. La figure 16 illustre cette méthode. Le signal de mesure de distance obtenu à partir des capteurs est représenté dans la première partie de la figure dans une fenêtre temporelle de cinq minutes. Ici, la valeur cent signifie que la mandibule est complètement fermée et le zéro signifie quant à lui que la mandibule est grande ouverte.

Le signal des événements de fermetures brutales (SCEvent - Sudden Closing Event) est un signal obtenu en calculant la différence de moyenne du signal de mesure. Le signal SCEvent n'est que la partie positive des résultats de la différence de moyenne mettant en évidence les endroits où la mandibule est fermée brutalement. L'amplitude de se signal dérivé révèle la grandeur de cette fermeture. Ce signal est, par conséquent, un marqueur d'événement de fermetures brutales.

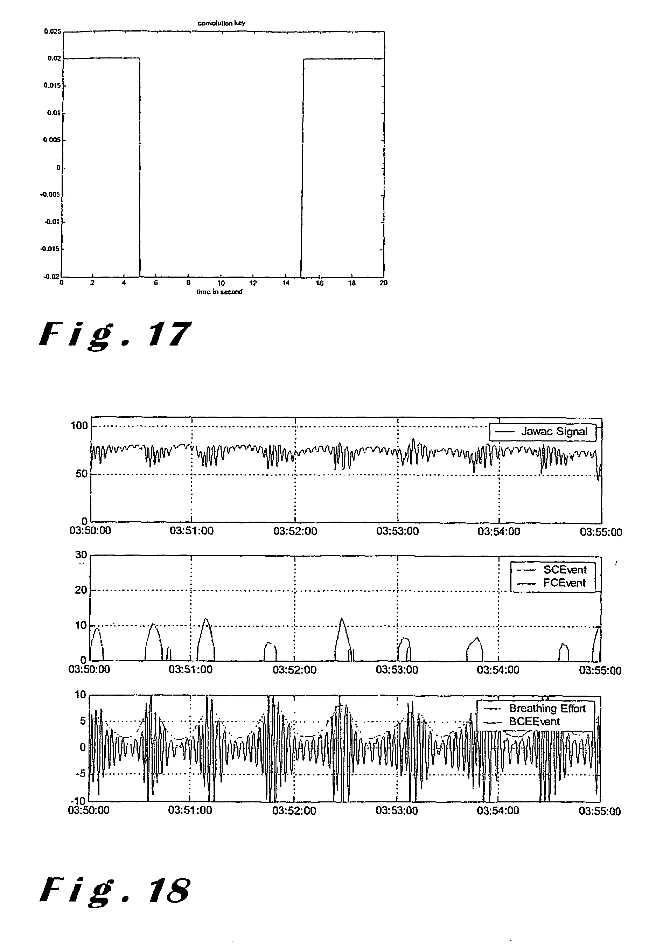

Le signal des événements de fermetures souples (FCEvent - Fluid Closing Event) est un signal obtenu par convolution du signal de mesure et d'une clé de convolution. Cette clé de convolution est reprise à la figure 17. L'objectif de cette convolution est de mettre en évidence les variations souples du signal de mesure qui représentent une ouverture de la mandibule suivie par une fermeture opposée et similaire. Le résultat de la convolution est repris à la figure 18 (FCEvent).

Le dernier signal obtenu par filtrage est le signal BCEvent. Nous filtrons tout d'abord le signal de mesure avec un filtre passe bande dans la bande de fréquence de la respiration (typiquement 0.2 à 0.4 Hz). Le résultat du filtrage est ce qui est appelé le signal d'effort respiratoire (Breathing Effort). Dès lors, le signal BCEvent est simplement l'enveloppe supérieure du signal d'effort respiratoire. La totalité de ces trois signaux dérivés sont les marqueurs d'une fin possible d'un événement apnéique.

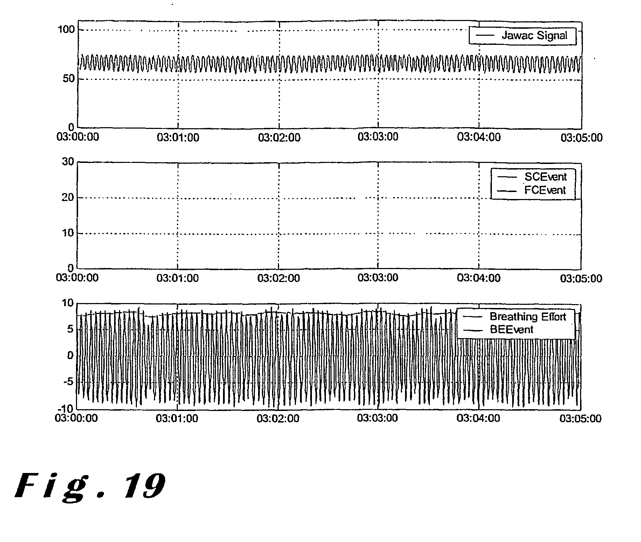

Le second objectif de cet étage A (figure14) est de détecter les périodes de ronflement. Le ronflement est défini comme un signal sinusoïdale oscillant à la fréquence de la respiration (typiquement 0.2 ... 0.4 Hz) et d'amplitude constante. La figure 19 représente une période de ronflement.

L'étape suivante de l'algorithme est utilisée pour délimiter tous les événements apnées candidats. La durée d'une apnée est généralement définie dans une fenêtre temporelle de 10 à 90 secondes. Les trois signaux dérivés (SCEvent, FCEvent, BEEvent) sont tout d'abord utilisés pour rejeter les événements qui ne représentent pas des apnées. Un événement est accepté si une des deux conditions suivantes est remplie :

- La présence de SCEvent et BEEvent dans la fenêtre temporelle considérée.
- La présence de FCEvent et BEEvent dans la fenêtre temporelle considérée.

Ensuite, pour tous les événements acceptés, il est regardé en arrière dans la fenêtre temporelle considérée pour chercher où l'apnée est supposée avoir débuté. Typiquement, l'apnée commence quand l'événement BBEvent commence à augmenter dans la période de temps considérée. C'est le cas pour les hyponées et les apnées obstructives. Dans le cas des apnées centrales ou mixtes, l'apnée commence avec aucun effort respiratoire et l'événement BBEvent est, dans ce cas, la signature de la fin de l'apnée. Il faut, par conséquent, dériver un autre signal de manière à délimiter les apnées centrales et mixtes. Dans la période temporelle considérée, on marque les endroits où l'effort respiratoire est nul. Le signal des événements NEBEvent pointe l'absence d'effort respiratoire en début d'apnée.

Le dernier signal dérivé est utilisé pour la détection des syndromes de résistance accrue de la voie aérienne supérieure (SRAVAS ou en anglais UARS - upper airway résistance syndrome): Dans la fenêtre temporelle considérée d'une apnée, nous marquons si l'ouverture moyenne de la mandibule est linéaire durant la totalité de l'apnée.

Le second aspect important de cette étape est de trouver les portions de signal où un des trois événements ci-dessus se répète avec une période temporelle stricte. La périodicité d'un événement sera utilisée pour confirmer la présence d'une salve d'apnée.

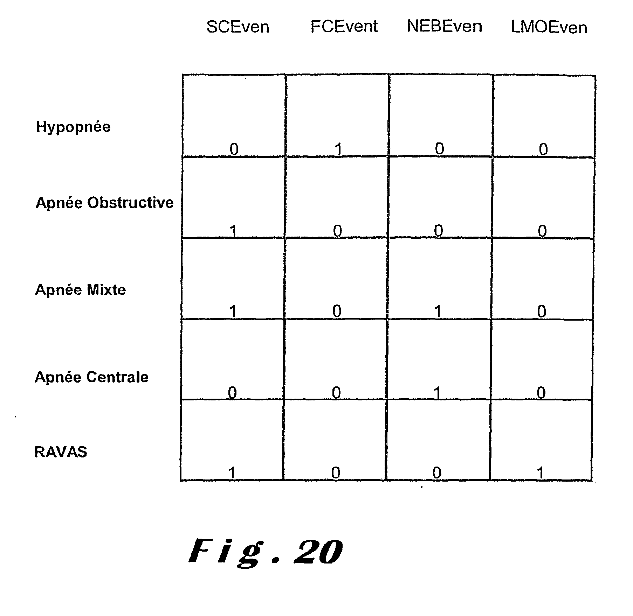

Le but de cette étape est de classifier toutes les apnées délimitées. Nous utilisons la table binaire suivante. Pour chaque apnée délimitée, le type d'apnée est caractérisé par la présence ou l'absence de tous les événements dans la région délimitée. Le tableau repris à la figure 20 illustre ceci.

Deux règles sont utilisées pour confirmer la classification ci-dessus. La classification ci-dessus peut contenir des grapho-éléments qui ne sont pas à proprement parlé des apnées. De manière à obtenir une spécificité et une sensibilité suffisante de l'algorithme, on accepte uniquement les grapho-éléments qui satisfont au moins une des deux règles suivantes :

Règle 1: Toutes les apnées détectées dans une région 'périodique' seront acceptées.
Règle 2: Chaque grapho-élément classé qui contient un événement BEEvent supérieur à un seuil spécifié sera accepté. Ici, les apnées isolées qui sont détectées mais qui n'ont pas un effort respiratoire suffisant seront rejetées.

De façon similaire à la détection des troubles respiratoires du sommeil, on utilise les capteurs pour réguler la pression d'un appareil à pression d'air positif et continu (continuous positive air pressure apparatus).

Le principe de la méthode proposée est le suivant :

1) On collecte les données dans un tampon. La longueur de celui-ci est définie de manière à contenir au moins une apnée. (typiquement 10 à 90 s).
2) On vérifie tout d'abord que la bouche est plus ou moins fermée (typiquement 80%).
3) Si la bouche est ouverte, on vérifie la présence des événements suivants : BEEvent, SCEvent et FCEvent. La périodicité est également vérifiée pour confirmation. Si un tel événement est observé, on augmente la pression et attend l'ensemble de données suivantes.
4) Si la bouche est plus ou moins fermée (habituellement 85 % de fermeture), On donne la possibilité d'utiliser une échelle temporelle différente pour diminuer la pression. La diminution de pression est plus lente que l'augmentation. Ici, on considère que la diminution de pression est dix fois plus lente que l'augmentation. Le rapport entre l'augmentation et la diminution peut être adapté pour chaque personne.

Le même détecteur de distance peut être utilisé dans toute autre application nécessitant la surveillance ou l'analyse du mouvement d'une articulation, en plaçant les deux éléments du capteur de part et d'autre de celle-ci.

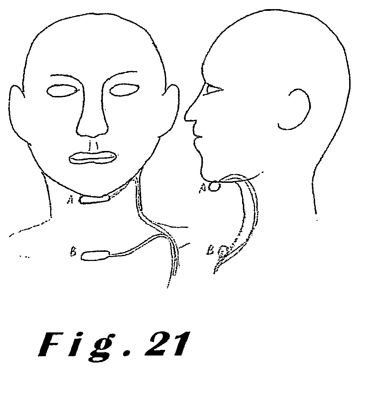

Le suivi de l'évolution de la maladie de Parkinson chez un patient en est un exemple. Dans ce cadre, il est utile d'enregistrer durant un ou plusieurs jours, l'état du patient pour détecter des situations de tremblement, de figement et de repliement. A cet effet, le capteur de distance peut être placé au niveau du menton et du thorax, comme indiqué à la figure 21, afin de mesurer les caractéristiques des mouvements de la tête. Des statistiques peuvent alors être établies pour juger de l'efficacité d'un traitement, par exemple. Alternativement, les capteurs peuvent être placés au coude, à la hanche, ou au genou avec des effets similaires.

- L'état de figement est détecté par une analyse de variabilité du signal sur des périodes données. On peut par exemple procéder par calcul de la variance du signal. Une variance de faible valeur est représentative d'un état de figement.
- L'état de repliement est un état de figement où la valeur moyenne de distance mesurée est de plus, très faible.
- Un état de tremblement peut être détecté par une analyse spectrale du signal. L'apparition d'une composante spectrale prédominante dans le signal de distance sur une période donnée est représentative d'un état de tremblement.

Le mouvement périodique des membres est une autre maladie du sommeil. L'utilisation du même détecteur de distance pour la détection de ces mouvements est possible. Le principe est tout à fait semblable au cas du suivi de la maladie de Parkinson. Toutefois, l'analyse recherche plutôt des mouvements isolés et leur périodicité plutôt que des états généraux.

La littérature abonde sur les moyens de détecter la perte de vigilance d'une personne, notamment durant la conduite d'un véhicule. Le capteur de distance présenté ici est utilisé pour de telles mesures selon deux modalités:

a. Mesure du dodelinement de la tête L'apparition d'un état de somnolence s'accompagne d'un relâchement des muscles du cou qui se traduit, en position verticale, par un abaissement progressif de la tête. Lorsque la personne en prend conscience, elle réagit en redressant brusquement la tête. Le placement du capteur de distance sur le menton et sur le thorax, conformément à la figure 21, permet de détecter ces événements. L'analyseur procède donc par repérage des mouvements brusques vers le haut, ce qui constitue un cas particulier de l'analyse.
b. Mesure de l'ouverture de la paupière

La miniaturisation des composants permet de placer un des éléments du capteur de distance sur la paupière, l'autre étant placé sous ou au-dessus de l'oeil. Lors d'une perte progressive de vigilance, de battements de paupières plus fréquents sont observés, de même qu'une diminution progressive de l'ouverture moyenne de la paupière. La détection de ces événements constitue également un cas particulier de l'analyse.

Des applications de sécurité nécessitent le contrôle du déclenchement d'une machine ou d'un dispositif en fonction de la distance, de telle manière à éviter une fausse manoeuvre ou éviter un usage intempestif. L'utilisation de ce capteur de distance à excitation ponctuelle permet cette utilisation. Dans ce cas, l'interrupteur du circuit d'excitation peut servir à la demande de déclenchement de l'utilisateur, mais le déclenchement effectif ne sera réalisé que par un élément matériel ou logiciel de décision après vérification de la distance à laquelle se trouve le dispositif de déclenchement. Un déclenchement de différents dispositifs en fonction de la distance est également possible, par exemple, mais non seulement, pour afficher des messages d'avertissement différents.

## Revendications

1. Dispositif de mesure de distance comprenant un émetteur et un récepteur, ledit émetteur étant agencé pour produire un champ magnétique à l'aide d'un circuit résonant ayant une fréquence de résonance, ledit récepteur étant agencé pour capter à ladite fréquence de résonance le champ magnétique émis par l'émetteur et convertir l'intensité du champ magnétique capté en un premier signal ayant une valeur d'énergie, ledit émetteur étant agencé pour produire ledit champ magnétique par intermittence, chaque émission ayant une énergie prédéterminée, ledit récepteur étant connecté à un détecteur agencé pour déterminer, un signal de mesure de distance représentant la distance entre l'émetteur et le récepteur, **caractérisé en ce que** ledit détecteur est agencé pour déterminer ledit signal de mesure de distance par corrélation dudit premier signal avec un deuxième signal prédéterminé ayant une forme d'onde représentative d'un signal à capter par le récepteur, ledit deuxième signal comportant une fenêtre temporelle ayant une durée prédétermine et comprenant au moins une sous-période initiale, une sous-période intermédiaire et une sous-période finale, ledit deuxième signal étant un signal alternatif synchronisé avec le premier signal et dont l'amplitude est atténuée durant les périodes initiales et finales et substantiellement au maximum durant la période intermédiaire.

2. Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant le signal à capter tel qu'obtenu en l'absence de perturbation.

3. Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant une forme d'onde sinusoïdale en synchronisation avec le premier signal elle-même multipliée par une fenêtre de Tukey à coefficient d'effilage réduit.

4.  Dispositif de mesure de distance suivant la revendication 1, **caractérisé en ce que** ledit détecteur est agencé pour réaliser ladite corrélation par multiplication et intégration avec ledit deuxième signal, lequel deuxième signal est formé par ladite forme d'onde représentant une forme d'onde carrée en synchronisation avec le premier signal.

5.  Dispositif de mesure de distance suivant l'une des revendications 1 à 4, **caractérisé en ce que** ledit émetteur est logé dans un boîtier et agencé pour produire ledit champ magnétique à l'extérieur dudit boîtier avec une puissance inférieure à 1mTesla, de préférence inférieure à 1 $\mu$Tesla.

6.  Dispositif de mesure de distance suivant l'une des revendications 1 à 5, **caractérisé en ce que** ledit émetteur comporte une inductance et un condensateur connectés en série entre eux et raccordés à l'aide de conducteurs électriques à un circuit d'excitation, ledit circuit d'excitation comprenant une source de tension et une résistance connectées, au travers desdits conducteurs électriques, en série avec l'inductance et le condensateur, ledit circuit d'excitation comprenant également un élément de commutation permettant de connecter entre eux lesdits conducteurs électriques.

7.  Dispositif de mesure de distance suivant la revendication 6, **caractérisé en ce que** ledit circuit d'excitation est placé à proximité de l'inductance et du condensateur et forme une unité autonome par rapport au récepteur.

8.  Détecteur de troubles du sommeil comprenant un dispositif de mesure de distance suivant l'une des revendications 1 à 7, **caractérisé en ce que** ledit dispositif est monté sur un support agencé pour être appliqué sur la tête d'un être vivant de telle façon à mesurer des mouvements de la bouche.

9.  Détecteur de troubles du sommeil suivant la revendication 8, **caractérisé en ce que** ledit détecteur comporte un analyseur ayant une entrée reliée au dispositif et agencée pour recevoir ladite mesure de distance, ledit analyseur étant agencé pour fractionner ledit signal de mesure de distance et appliquer des fenêtres temporelles sur chaque fraction du signal de mesure de distance ainsi obtenu, ledit détecteur comprenant également une mémoire pour stocker une série de formes de signaux caractérisant dans une fenêtre temporelle des mouvements de la bouche d'un être vivant, ledit analyseur étant agencé pour comparer lesdites fractions du signal de mesure de distance à chacune desdites formes de la série et pour produire un signal de détection en cas de correspondance entre ladite fraction et ladite forme, le signal de détection comprenant également un indicateur indiquant la forme ayant menée à ladite correspondance.

10.  Détecteur de troubles du sommeil suivant la revendication 9, **caractérisé en ce que** ladite série comporte une première forme indiquant une fermeture soudaine de la bouche, une deuxième forme indiquant une lente ouverture de la bouche suivie d'une lente fermeture de la bouche ainsi qu'une troisième forme indiquant un accroissement de l'amplitude du signal à la fréquence de respiration suivie d'une décroissance du signal à la fréquence de respiration.

11.  Détecteur de troubles du sommeil suivant la revendication 9, **caractérisé en ce que** ledit analyseur est agencé pour produire un signal d'apnée lorsque pour une même fenêtre le signal de détection indique à la fois une première et une troisième forme ou indique à la fois une deuxième et une troisième forme.

12.  Détecteur de troubles du sommeil suivant l'une des revendications 9 à 11, **caractérisé en ce que** ladite série comporte une quatrième forme indiquant un ronflement.

13.  Détecteur de troubles du sommeil suivant l'une des revendications 9 à 12, **caractérisé en ce que** ledit détecteur comporte un analyseur ayant une entrée reliée au dispositif et agencée pour recevoir ledit signal de mesure de distance, ledit analyseur étant agencé pour repérer dans ledit signal de mesure de distance des formes de signaux représentant des événements brefs et récurrents et pour produire un signal de détection à chaque occurrence de tels signaux, le signal de détection comprenant également un indicateur indiquant la forme ayant menée audit signal de détection.

14.  Détecteur de troubles du sommeil suivant la revendication 13, **caractérisé en ce qu'**il comporte un élément de décision utilisant ledit signal de détection pour fournir une indication de traitement insuffisant, correct ou exagéré des troubles du sommeil visés.

**15.** Analyseur de mouvement comprenant un dispositif de mesure de distance suivant l'une des revendications 1 à 8, **caractérisé en ce que** ledit dispositif est monté sur un support agencé pour être appliqué autour d'une articulation d'un être vivant de telle façon à mesurer les caractéristiques et/ou les statistiques des mouvements de cette articulation.

**16.** Détecteur de mouvements périodiques des membres en cours de sommeil comprenant un analyseur de mouvement suivant la revendication 15, **caractérisé en ce que** ledit analyseur est agencé pour repérer dans ledit signal de mesure de distance des formes de signaux représentant des événements brefs et récurrents et pour produire un signal de détection à chaque occurrence de tels signaux, le signal de détection comprenant également un indicateur indiquant la forme ayant menée audit signal de détection.

**17.** Appareillage de suivi de l'évolution de la maladie de parkinson comprenant un analyseur de mouvement suivant la revendication 15, **caractérisé en ce que** ledit analyseur est agencé pour repérer dans ledit signal de mesure de distance des formes de signaux représentant des états de tremblement, de figement et de repliement pour produire un signal de détection, le signal de détection comprenant également un indicateur indiquant la forme ayant menée audit signal de détection.

**18.** Appareillage de détection d'une perte de vigilance comprenant un analyseur de mouvement suivant la revendication 15, **caractérisé en ce que** ledit dispositif de mesure est placé de telle manière à mesurer l'inclinaison de la tête et ledit analyseur est agencé pour repérer dans ledit signal de mesure de distance des événements d'inclinaison lente et relève brutale de la tête, pour produire un signal de détection.

**19.** Appareillage de détection d'une perte de vigilance comprenant un analyseur de mouvement suivant la revendication 15, **caractérisé en ce que** ledit dispositif de mesure est agencé de telle manière à mesurer l'amplitude d'ouverture des paupières et ledit analyseur est agencé pour repérer dans ledit signal de mesure de distance des événements de clignement récurrent des paupières et un état de diminution progressive de l'amplitude moyenne d'ouverture des paupières, pour produire un signal de détection.

**Claims**

**1.** Distance measuring device comprising an emitter and a receiver, said emitter being arranged to produce a magnetic field by means of a resonant circuit having a resonant frequency, said receiver being arranged to pick up at said resonant frequency the magnetic field emitted by the emitter and convert the strength of the magnetic field picked up into a first signal having an energy value, said emitter being arranged to produce said magnetic field intermittently, each emission having a predetermined energy, said receiver being connected to a detector arranged to determine a distance measurement signal representing the distance between the emitter and the receiver, **characterised in that** said detector is arranged to determine said distance measurement signal by correlation of said first signal with a second predetermined signal having a waveform representative of a signal to be picked up by the receiver, said second signal comprising a time window having a predetermined duration and comprising at least an initial sub-period, an intermediate sub-period and a final sub-period, said second signal being an alternating signal synchronized with the first signal and whereof the amplitude is attenuated during the initial and final periods and substantially at a maximum during the intermediate period.

**2.** Distance measuring device as claimed in claim 1, **characterized in that** said detector is arranged to implement said correlation by multiplication and integration with said second signal, which second signal is formed by said waveform representing the signal to be picked up as obtained in the absence of perturbation.

**3.** Distance measuring device as claimed in claim 1, **characterized in that** said detector is arranged to implement said correlation by multiplication and integration with said second signal, which second signal is formed by said waveform representing a sinusoidal waveform in synchronization with the first signal itself multiplied by a Tukey window with reduced taper factor.

**4.** Distance measuring device as claimed in claim 1, **characterised in that** said detector is arranged to implement said correlation by multiplication and integration with said second signal, which second signal is formed by said waveform representing a square waveform in synchronization with the first signal.

**5.** Distance measuring device as claimed in one of claims 1 to 4, **characterized in that** said emitter is housed in a case and arranged to produce said magnetic field outside said case with a power less than 1 mTesla, preferably less than 1 μTesla.

6. Distance measuring device as claimed in one of claims 1 to 5, **characterized in that** said emitter comprises an inductance and a capacitor connected in series with one another and connected by means of electrical conductors to an energizing circuit, said energizing circuit comprising a voltage source and a resistor connected, through said electrical conductors, in series with the inductance and the capacitor, said energizing circuit also comprising a switching element making it possible to connect said electrical conductors to one another.

7. Distance measuring device as claimed in claim 6, **characterized in that** said energizing circuit is placed close to the inductance and the capacitor and forms an autonomous unit with respect to the receiver.

8. Sleep disorder detector comprising a distance measuring device as claimed in one of claims 1 to 7, **characterized in that** said device is mounted on a support arranged to be applied onto the head of a living being so as to measure movements of the mouth.

9. Sleep disorder detector as claimed in claim 8, wherein said detector comprises an analyzer having an input connected to the device and arranged to receive said distance measurement, said analyzer being arranged to divide said distance measurement signal into fractional parts and apply time windows to each fractional part of the distance measurement signal thus obtained, said detector also comprising a memory for storing a series of signal forms **characterizing in** a time window movements of the mouth of a living being, said analyzer being arranged to compare said fractional parts of the distance measurement signal with each of said forms of the series and to produce a detection signal in the event of correspondence between said fractional part and said form, the detection signal also comprising an indicator indicating the form having led to said correspondence.

10. Sleep disorder detector as claimed in claim 9, **characterised in that** said series comprises a first form indicating a sudden closing of the mouth, a second form indicating a slow opening of the mouth followed by a slow closing of the mouth, and a third form indicating an increase in the amplitude of the signal at the breathing frequency followed by a decrease in the signal at the breathing frequency.

11. Sleep disorder detector as claimed in claim 9, **characterized in that** said analyzer is arranged to produce an apnea signal when for the same window the detection signal indicates both a first and a third form or indicates both a second and a third form.

12. Sleep disorder detector as claimed in one of claims 9 to 11, **characterized in that** said series comprises a fourth form indicating snoring.

13. Sleep disorder detector as claimed in one of claims 9 to 12, **characterized in that** said detector comprises an analyzer having an input connected to the device and arranged to receive said distance measurement signal, said analyzer being arranged to identify, in said distance measurement signal, signal forms representing brief and recurrent events and to produce a detection signal at each occurrence of such signals, the detection signal also comprising an indicator indicating the form having led to said detection signal.

14. Sleep disorder detector as claimed in claim 13, this detector comprising a decision element using said detection signal to provide an indication of insufficient, correct or excessive treatment for the targeted sleep disorders.

15. Movement analyzer comprising a distance measuring device as claimed in one of claims 1 to 8, **characterized in that** said device is mounted on a support arranged to be applied around a joint of a living being so as to measure the characteristics and/or statistics of the movements of this joint.

16. Detector of periodic movements of limbs during sleep comprising a movement analyzer as claimed in claim 15, **characterized in that** said analyzer is arranged to identify, in said distance measurement signal, signal forms representing brief and recurrent events and to produce a detection signal at each occurrence of such signals, the detection signal also comprising an indicator indicating the form having led to said detection signal.

17. Equipment for monitoring the development of Parkinson's disease comprising a movement analyzer as claimed in claim 15, **characterised in that** said analyzer is arranged to identify, in said distance measurement signal, signal forms representing tremor, rigidity and stooping states in order to produce a detection signal, the detection signal also comprising an indicator indicating the form having led to said detection signal.

18. Equipment for detecting a loss of vigilance comprising a movement analyzer as claimed in claim 15, **characterized in that** said measuring device is placed so as to measure the inclination of the head and said analyzer is arranged to identify, in said distance measurement signal, events of slow inclination and sudden lifting of the head, in order to produce a detection signal.

19. Equipment for detecting a loss of vigilance comprising a movement analyzer as claimed in claim 15, **characterized in that** said measuring device is arranged so as to measure the amplitude of opening of the eyelids and said analyzer is arranged to identify, in said distance measurement signal, events of recurrent blinking of the eyelids and a state of progressive decrease in the mean amplitude of opening of the eyelids, in order to produce a detection signal.


**Patentansprüche**

1. Abstandsmessvorrichtung, umfassend einen Sender und einen Empfänger, wobei der Sender derart angeordnet ist, dass er ein Magnetfeld mit Hilfe einer Resonanzschaltung mit einer Resonanzfrequenz erzeugt, wobei der Empfänger derart angeordnet ist, dass er auf der Resonanzfrequenz das vom Sender entsandte Magnetfeld erfasst und die Stärke des erfassten Magnetfeldes in ein erstes Signal mit einem Energiewert umformt, wobei der Sender derart angeordnet ist, dass er das Magnetfeld intermittierend erzeugt, wobei jede Entsendung eine vorbestimmte Energie hat, wobei der Empfänger an einem Detektor angeschlossen ist, der derart angeordnet ist, dass er ein Abstandsmesssignal, das den Abstand zwischen dem Sender und dem Empfänger darstellt, bestimmt, **dadurch gekennzeichnet, dass** der Detektor derart angeordnet ist, dass er das Abstandsmesssignal durch Korrelation des ersten Signals mit einem vorbestimmten zweiten Signal bestimmt, das eine Wellenform hat, die für ein vom Empfänger zu erfassendes Signal repräsentativ ist, wobei das zweite Signal ein Zeitfenster mit einer vorbestimmten Dauer, umfassend mindestens eine Ausgangsunterperiode, eine Zwischenunterperiode und eine Endunterperiode, umfasst, wobei das zweite Signal ein mit dem ersten Signal synchronisiertes Wechselsignal ist, dessen Amplitude während der Ausgangs- und Endperioden und im Wesentlichen maximal während der Zwischenperiode gedämpft wird.

2. Abstandsmessvorrichtung nach Anspruch 1, **dadurch gekennzeichnet, dass** der Detektor derart angeordnet ist, dass er die Korrelation durch Multiplikation und Integration mit dem zweiten Signal durchführt, wobei das zweite Signal von der Wellenform gebildet ist, die das zu erfassende Signal, wie bei Nichtvorhandensein einer Störung erhalten, darstellt.

3. Abstandsmessvorrichtung nach Anspruch 1, **dadurch gekennzeichnet, dass** der Detektor derart angeordnet ist, dass er die Korrelation durch Multiplikation und Integration mit dem zweiten Signal durchführt, wobei das zweite Signal von der Wellenform gebildet ist, die eine Sinuswellenform in Synchronisation mit dem ersten Signal darstellt, welches selbst durch ein Tukey-Fenster mit verringertem Zuspitzungskoeffizienten multipliziert wird.

4. Abstandsmessvorrichtung nach Anspruch 1, **dadurch gekennzeichnet, dass** der Detektor derart angeordnet ist, dass er die Korrelation durch Multiplikation und Integration mit dem zweiten Signal durchführt, wobei das zweite Signal von der Wellenform gebildet ist, die eine quadratische Wellenform in Synchronisation mit dem ersten Signal darstellt.

5. Abstandsmessvorrichtung nach einem der Ansprüche 1 bis 4, **dadurch gekennzeichnet, dass** der Sender in einem Gehäuse untergebracht und derart angeordnet ist, dass er das Magnetfeld außerhalb des Gehäuses mit einer Leistung unter 1mTesla, vorzugsweise unter 1µTesla, erzeugt.

6. Abstandsmessvorrichtung nach einem der Ansprüche 1 bis 5, **dadurch gekennzeichnet, dass** der Sender eine Induktanz und einen Kondensator umfasst, die in Serie miteinander verbunden und mit Hilfe von elektrischen Leitern an eine Erregerschaltung angeschlossen sind, wobei die Erregerschaltung eine Spannungsquelle und einen Widerstand umfasst, die über die elektrischen Leiter in Serie mit der Induktanz und dem Kondensator geschaltet sind, wobei die Erregerschaltung auch ein Schaltelement umfasst, die es ermöglicht, die elektrischen Schalter miteinander zu verbinden.

7. Abstandsmessvorrichtung nach Anspruch 6, **dadurch gekennzeichnet, dass** die Erregerschaltung in der Nähe der Induktanz und des Kondensators angeordnet ist und eine autonome Einheit im Bezug zum Empfänger bildet.

**8.** Detektor für Schlafstörungen, umfassend eine Abstandsmessvorrichtung nach einem der Ansprüche 1 bis 7, **dadurch gekennzeichnet, dass** die Vorrichtung auf einem Träger montiert ist, der derart angeordnet ist, dass er am Kopf eines Lebewesens angebracht werden kann, um Bewegungen des Mundes zu messen.

**9.** Detektor für Schlafstörungen nach Anspruch 8, **dadurch gekennzeichnet, dass** der Detektor einen Analysator mit einem Eingang umfasst, der an die Vorrichtung angeschlossen und derart angeordnet ist, dass er die Abstandsmessung empfängt, wobei der Analysator derart angeordnet ist, dass er das Abstandsmesssignal teilt und Zeitfenster an jeden Bruchteil des so erhaltenen Abstandsmesssignals anlegt, wobei der Detektor auch einen Speicher umfasst, um eine Reihe von Signalformen zu speichern, die in einem Zeitfenster Bewegungen des Mundes eines Lebewesens kennzeichnen, wobei der Analysator derart angeordnet ist, dass er Bruchteile des Abstandsmesssignals mit jeder der Formen der Reihe vergleicht und ein Erfassungssignal im Falle einer Übereinstimmung zwischen dem Bruchteil und der Form erzeugt, wobei das Erfassungssignal auch einen Indikator umfasst, der die Form anzeigt, die zur Übereinstimmung geführt hat.

**10.** Detektor für Schlafstörungen nach Anspruch 9, **dadurch gekennzeichnet, dass** die Reihe eine erste Form umfasst, die ein plötzliches Schließen des Mundes anzeigt, eine zweite Form, die ein langsames Öffnen des Mundes, gefolgt von einem langsamen Schließen des Mundes anzeigt, sowie eine dritte Form umfasst, die eine Vergrößerung der Amplitude des Signals bei der Atemfrequenz, gefolgt von einer Verringerung des Signals bei der Atemfrequenz anzeigt.

**11.** Detektor für Schlafstörungen nach Anspruch 9, **dadurch gekennzeichnet, dass** der Analysator derart angeordnet ist, dass er ein Atemstillstandssignal erzeugt, wenn das Erfassungssignal für ein selbes Fenster gleichzeitig eine erste und eine dritte Form oder gleichzeitig eine zweite und eine dritte Form anzeigt.

**12.** Detektor für Schlafstörungen nach einem der Ansprüche 9 bis 11, **dadurch gekennzeichnet, dass** die Reihe eine vierte Form umfasst, die ein Schnarchen anzeigt.

**13.** Detektor für Schlafstörungen nach einem der Ansprüche 9 bis 12, **dadurch gekennzeichnet, dass** der Detektor einen Analysator mit einem Eingang umfasst, der an die Vorrichtung angeschlossen und derart angeordnet ist, dass er in dem Abstandsmesssignal Signalformen erfasst, die kurze und rückläufige Ereignisse darstellen, und ein Erfassungssignal bei jedem Auftreten derartiger Signale erzeugt, wobei das Erfassungssignal auch einen Indikator umfasst, der die Form anzeigt, die zum Erfassungssignal geführt hat.

**14.** Detektor für Schlafstörungen nach Anspruch 13, **dadurch gekennzeichnet, dass** er ein Entscheidungselement umfasst, das das Erfassungssignal verwendet, um eine Anzeige einer unzureichenden, richtigen oder übertriebenen Behandlung der betreffenden Schlafstörungen zu liefern.

**15.** Bewegungsanalysator, umfassend eine Abstandsmessvorrichtung nach einem der Ansprüche 1 bis 8, **dadurch gekennzeichnet, dass** die Vorrichtung auf einem Träger montiert ist, der derart angeordnet ist, dass er um ein Gelenk eines Lebewesens gelegt werden kann, um die Eigenschaften und/oder Statistiken der Bewegungen dieses Gelenks zu messen.

**16.** Detektor von periodischen Bewegungen der Gliedmaßen während des Schlafs, umfassend einen Bewegungsanalysator nach Anspruch 15, **dadurch gekennzeichnet, dass** der Analysator derart angeordnet ist, dass er in dem Abstandsmesssignal Signalformen erfasst, die kurze und rückläufige Ereignisse darstellen, und ein Erfassungssignal bei jedem Auftreten solcher Signale erzeugt, wobei das Erfassungssignal auch einen Indikator umfasst, der die Form anzeigt, die zum Erfassungssignal geführt hat.

**17.** Gerät zur Verfolgung der Entwicklung der Parkinson-Krankheit, umfassend einen Bewegungsanalysator nach Anspruch 15, **dadurch gekennzeichnet, dass** der Analysator derart angeordnet ist, dass er in dem Abstandsmesssignal Signalformen erfasst, die Zustände des Zitterns, Erstarrens und Vornüberneigens darstellen, um ein Erfassungssignal zu erzeugen, wobei das Erfassungssignal auch einen Indikator umfasst, der die Form anzeigt, die zum Erfassungssignal geführt hat.

**18.** Gerät zur Erfassung eines Wachsamkeitsverlusts, umfassend einen Bewegungsanalysator nach Anspruch 15, **dadurch gekennzeichnet, dass** die Messvorrichtung derart angeordnet ist, dass sie die Neigung des Kopfes misst, und dass der Analysator derart angeordnet ist, dass er in dem Abstandsmesssignal Ereignisse eines langsamen Neigens und plötzliche Hebens des Kopfes erfasst, um ein Erfassungssignal zu erzeugen.

**19.** Gerät zur Erfassung eines Wachsamkeitsverlusts, umfassend einen Bewegungsanalysator nach Anspruch 15, **dadurch gekennzeichnet, dass** die Messvorrichtung derart angeordnet ist, dass sie die Amplitude des Öffnens der Augenlider misst, und dass der Analysator derart angeordnet ist, dass er in dem Abstandsmesssignal Ereignisse eines rückläufigen Schlagens der Augenlider und einen Zustand der fortschreitenden Verringerung der durchschnittlichen Öffnungsamplitude der Augenlider erfasst, um ein Erfassungssignal zu erzeugen.

**Fig. 1**

Temps (unités arbitraires)

**Fig. 2**

**Fig. 3**

**Fig. 4**

Signal
du détecteur

20

21

Modèle

Valeur
de mesure

**Fig. 5**

Temps (unités arbitraires)

**Fig. 6**

Temps (unités arbitraires)

**Fig. 7**

**Fig. 8**

**Fig. 9**

*F i g . 10*

*Fig. 11*

**Fig. 12**

**Fig. 13**

**Filtre Eveil/Sommeil**

A

**Filtre Ronflement**

**Filtre Fin d'Apnée**

B

**Délimitation de Patterns**

**Evaluation de la périodicité des fins d'apnées**

C

**Classification des Patterns**

D

**Confirmation des Patterns**

*Fig. 14a*

**F i g . 14b**

**Fig. 15**

**Fig. 16**

*Fig. 17*

*Fig. 18*

*Fig. 19*

|  | SCEven | FCEvent | NEBEven | LMOEven |
|---|---|---|---|---|
| Hypopnée | 0 | 1 | 0 | 0 |
| Apnée Obstructive | 1 | 0 | 0 | 0 |
| Apnée Mixte | 1 | 0 | 1 | 0 |
| Apnée Centrale | 0 | 0 | 1 | 0 |
| RAVAS | 1 | 0 | 0 | 1 |

# Fig. 20

**Fig. 21**

Forme des filtres de détection

Fig. 22

Spectre d'amplitude des filtres équivalents de réception

Fréquence (Hz)

# Fig. 23

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

* DE 4114398


**Littérature non-brevet citée dans la description**

* **Harris, F.J.** On the use of windows for harmonic analysis with the discrete Fourier transform. *Proceedings of the IEEE.,* Janvier 1978, vol. 66, 66-67